(19) Europäisches Patentamt
European Patent Office
Office européen des brevets

(11)  EP 1 713 570 B1

(12)  FASCICULE DE BREVET EUROPEEN

(45) Date de publication et mention
de la délivrance du brevet:
**04.07.2018  Bulletin 2018/27**

(21) Numéro de dépôt: **05717504.4**

(22) Date de dépôt: **27.01.2005**

(51) Int Cl.:
*B01F 3/08* *(2006.01)*        *B01F 17/00* *(2006.01)*

(86) Numéro de dépôt international:
**PCT/FR2005/000183**

(87) Numéro de publication internationale:
**WO 2005/082507 (09.09.2005 Gazette 2005/36)**

(54) **COMPOSITIONS D HUILE CRISTALLISABLE STABILISEES PAR DES PARTICULES SOLIDES COLLOI DALES**

DURCH FESTE KOLLOIDALE TEILCHEN STABILISIERTE ÖLZUSAMMENSETZUNGEN

CRYSTALLISABLE OIL COMPOSITIONS STABILISED BY SOLID COLLOIDAL PARTICLES

(84) Etats contractants désignés:
**AT BE BG CH CY CZ DE DK EE ES FI FR GB GR
HU IE IS IT LI LT LU MC NL PL PT RO SE SI SK TR**

(30) Priorité:  **27.01.2004  FR 0400761**

(43) Date de publication de la demande:
**25.10.2006  Bulletin 2006/43**

(73) Titulaire: **CENTRE NATIONAL DE LA RECHERCHE
SCIENTIFIQUE (C.N.R.S.)
75016 Paris (FR)**

(72) Inventeurs:
• **SCHMITT, Véronique
F-33400 Talence (FR)**
• **KAHN, Joanna
F-33850 Leognan (FR)**
• **RECULUSA, Stéphane
Résidence Campus Haut Brion
F-33600 Pessac (FR)**

• **RAVAINE, Serge
F-33610 Cestas (FR)**
• **LEAL CALDERON, Fernando
F-33650 La Brede (FR)**
• **ARDITTY, Stéphane
Résidence Villebon
F-91140 VIllebon sur Yvette (FR)**

(74) Mandataire: **Lavoix
2, place d'Estienne d'Orves
75441 Paris Cedex 09 (FR)**

(56) Documents cités:
EP-A- 0 342 134          EP-A- 0 987 002
WO-A-00/07547          WO-A-94/17779
WO-A-03/028676          FR-A- 2 852 964
GB-A- 487 855          GB-A- 519 769
GB-A- 666 617          US-A- 4 610 944
US-B1- 6 245 850

**Description**

La présente invention concerne des compositions stables comprenant une phase aqueuse continue et une phase lipidique dispersée à base d'huile cristallisable.

Les compositions comprenant une phase lipidique solide dispersée dans une phase aqueuse sont intéressantes notamment pour la préparation de compositions cosmétiques, pharmaceutiques ou alimentaires. Elles peuvent également être utiles dans le domaine des revêtements de surface tels que les peintures ou les adhésifs.

Dans ces applications, il est généralement souhaité que ces compositions soient stables dans le temps, c'est-à-dire qu'elles présentent typiquement une stabilité au stockage de plusieurs mois.

Il est connu de stabiliser des émulsions par addition d'agents tensioactifs. Les agents tensioactifs possèdent une affinité à la fois pour la phase aqueuse et pour la phase lipidique et se concentrent donc à l'interface des deux phases. Ils jouent alors un rôle de stabilisateur en prévenant la recombinaison des gouttes de coagulation de la phase dispersée, empêchant ainsi la séparation de phases.

On connaît par ailleurs de S.U. Pickering, J. Chem. Soc. 91, pp 2001-2021 (1907) des émulsions comprenant une phase dispersée liquide, dites « Pickering », c'est-à-dire stabilisées seulement par des particules solides colloïdales. Par exemple, le document EP 0 987 002 A décrit une telle émulsion O/W du type « pickering », stabilisée par des particules colloïdales. La stabilisation de compositions comprenant une phase lipidique solide pose toutefois des problèmes particuliers. En effet, la phase dispersée solide, lorsqu'elle cristallise, prend généralement une forme anisotrope. La surface de la phase dispersée devient alors irrégulière et peut présenter des aspérités à l'échelle de plusieurs dizaines de nanomètres. Au contact de deux gouttes, les aspérités peuvent percer le film de phase continue séparant les gouttes car la pression de contact s'exerce sur la seule surface de la pointe de l'aspérité. Cette instabilité, aussi appelée « edge-piercing » ou coalescence partielle, conduit à une déstabilisation rapide de la composition à des températures inférieures ou égales à la température de cristallisation. Cette instabilité apparaît en particulier lorsque les compositions sont concentrées. Elles sont également fréquentes lors de l'application d'une telle composition sur un substrat solide.

Ainsi, il se révèle difficile de stabiliser ces compositions, et ce d'autant plus que la proportion de phase dispersée est élevée.

Or, la présence de tensioactifs ne permet généralement pas d'obtenir des compositions comprenant une phase lipidique solide dispersée présentant une stabilité suffisante.

Il existe en outre de nombreuses applications pour lesquelles la présence de tensioactifs à des concentrations élevées est indésirable. Ainsi, certains tensioactifs sont, aux concentrations utilisées, mal tolérés ou même nocifs pour le corps humain. Par ailleurs, ils peuvent poser des problèmes environnementaux. Enfin, on a pu observer une altération indésirable des propriétés de la composition, par exemple du caractère adhésif de la phase dispersée.

Aussi, le but de la présente invention est de fournir une composition comprenant une phase lipidique au moins partiellement solide dispersée dans une phase aqueuse qui soit stable et ne présente pas les inconvénients indiqués ci-dessus, un procédé permettant sa préparation et son utilisation pour la préparation de produits cosmétiques, pharmaceutiques ou alimentaires, peintures, adhésifs et revêtements.

Plus précisément, l'invention concerne, selon un premier aspect, une composition comprenant une phase aqueuse continue, une phase lipidique dispersée comprenant majoritairement une huile cristallisable, et des particules solides colloïdales à titre d'agent stabilisant, selon la revendication 1. La phase lipidique solide dispersée de la composition comprend de 50 à 100 %, en particulier de 90 à 100 % en poids d'huile cristallisable par rapport au poids total en phase lipidique.

On entend dans le cadre de cet exposé par le terme « huile cristallisable » un composé ou mélange de composés lipidiques dont le point de fusion est supérieur à 15°C, de préférence compris entre 35 et 50°C, et en particulier entre 40 et 45°C.

De préférence, la phase lipidique dispersée est majoritairement sous forme solide aux températures considérées. Elle peut toutefois également renfermer une proportion mineure de lipide à l'état liquide.

Ainsi, la phase lipidique dispersée est au moins partiellement, et tout particulièrement complètement, solide aux températures inférieures à 50°C et de préférence entre 15 et 35 °C, et tout particulièrement à température ambiante (20°C).

De préférence, la composition est cependant essentiellement exempte de phase gazeuse.

Avantageusement, la composition selon l'invention est stable au stockage et en cours d'utilisation, pour une fraction volumique de la phase lipidique allant jusqu'à 50% par rapport à la composition. De préférence, la composition selon l'invention comprend alors une fraction volumique en phase lipidique comprise entre 0,01% et 50%.

Avantageusement, la phase dispersée de la composition est monodisperse.

On entend par le terme « monodisperse » une distribution étroite de la taille de globules de la phase dispersée. Cette distribution est exprimée par l'indice de polydispersité, lequel est calculé de manière suivante :

$$U = \frac{1}{\overline{D}} \frac{\sum_i N_i D_i^3 \left| \overline{D} - D_i \right|}{\sum_i N_i D_i^3}$$

où Ni est le nombre total de globules de diamètre $D_i$ et $\overline{D}$ est le diamètre médian i.e. le diamètre pour lequel la fraction volumique cumulée est égale à 50%. Cette formule est appliquée dans les granulomètres de la société Malvern Instrument.

On entend par le terme « globules » les objets que forme la phase lipidique dispersée dans la composition. Selon la température, ce terme désigne des gouttelettes lorsque la phase lipidique est liquide et des particules lorsque la phase lipidique est solide.

Les globules de phase lipidique dispersée dans la composition présentent typiquement une taille moyenne en volume comprise entre 1 et 500 $\mu$m, de préférence entre 5 et 200$\mu$m.

Par ailleurs, la phase dispersée possède un indice de polydispersité inférieur à 40% et de préférence inférieur à 30%.

Selon l'invention, la composition est stabilisée grâce à la présence de particules solides colloïdales. De préférence, les particules solides colloïdales sont présentes essentiellement à l'interface formée entre la phase aqueuse continue et la phase lipidique dispersée de la composition.

La quantité nécessaire de particules solides pour la stabilisation dépend de la nature de la composition.

Généralement, une quantité de 0,01% à 10%, en particulier 0,1% à 5% en poids de particules solides colloïdales par rapport au poids de la composition est appropriée.

Avantageusement, la quantité de particules solides est choisie selon la taille de globules de la phase dispersée souhaitée, comme il apparaîtra plus loin.

Les particules solides colloïdales peuvent être minérales ou organiques. De préférence, il s'agit de particules minérales. Particulièrement préférées sont celles choisies dans le groupe des oxydes, hydroxydes et sulfates de métaux. Particulièrement préférés est les oxydes de silicium, de titane, de zirconium et de fer ou encore les silicates, et notamment les argiles. Enfin, on peut citer les particules colloïdales de carbone. Parmi ceux-ci, les particules d'oxydes de silicium ou d'oxydes de titane présentent l'avantage d'être aisément accessibles et peu coûteuses. Parmi les particules solides colloïdales organiques, on peut citer notamment les particules polymériques, par exemple des particules de latex.

Afin d'être colloïdales, les particules solides présentent généralement une taille inférieure au micromètre. De préférence, la taille des particules est supérieure à la dimension caractéristique des aspérités de la phase dispersée solide.

Ainsi, les particules solides présentent généralement une taille moyenne comprise entre 10 et 1000 nm, et de préférence entre 100 et 700 nm.

A titre d'exemple de ce type de particules, on peut citer le produit Aerosil 130 de chez Degussa, des particules de silice ayant un diamètre moyen de 21 nm.

On peut également citer les silices sensiblement sphériques de type Stöber (obtenues selon Stöber, W.; Fink, A.; Bohn, E. J. Coll. Interface Sci. 1968, 26, 62-69) dont la dispersion de taille est réduite ou encore des silices commercialisées sous la référence Seoster KEP50 par la société Shokubaï, sous le nom de Klébosol par la société Clariant ou sous le nom de Ludox par la société Aldrich.

Des particules de taille inférieure à 10 nm peuvent également être utilisées à condition d'être adsorbées en plusieurs couches ou bien sous forme d'agrégats, le but étant que les particules adsorbées forment une couche d'épaisseur supérieure à la dimension des aspérités. La présence de sel tel que le chlorure de sodium (NaCl) dans la phase continue peut favoriser la formation de tels agrégats.

Il est à noter que la simple présence de ces particules à l'interface, quelle que soit leur dimension, modifie le mode de cristallisation de la phase lipidique. Par exemple, en l'absence de particules colloïdales, la paraffine en . blocs de chez Merck cristallise partiellement sous forme de plaquettes hexagonales. Cela traduit la formation de monocristaux à l'échelle de chaque gouttelette. En revanche, les particules minérales constituent des sites de nucléation et l'objet formé n'est donc généralement pas un monocristal. De ce fait, l'objet final est moins anisotrope et présente une rugosité de surface réduite.

Par ailleurs, les particules solides colloïdales peuvent être fonctionnalisées en surface afin d'améliorer leur adsorption à l'interface de la composition. Les particules fonctionnalisées en surface par des composés comportant des groupements hydrophobes sont particulièrement préférées. A titre d'exemple de ce type de composés, on peut mentionner les trialkoxysilanes de formule R-Si-(OR')$_3$, dans laquelle R est un alkyle linéaire ou ramifié en $C_1$ à $C_{12}$, en particulier de $C_2$ à $C_{10}$ et tout particulièrement n-octyle, portant éventuellement un groupe amine, et R', identique ou différent, est un groupe alkyle linéaire ou ramifié en $C_1$ à $C_{12}$, en particulier de $C_1$ à $C_6$ et tout particulièrement éthyle.

Les particules peuvent aussi être fonctionnalisées par adsorption de molécules de tensioactif. En effet, les particules colloïdales solides présentent généralement une surface hydrophile et chargée, ce qui ne favorise pas l'adsorption des particules sur la phase lipidique dispersée.

Il peut alors, dans ces cas, être avantageux d'ajouter un tensioactif dans la composition. Le tensioactif permet de conférer une certaine hydrophobie aux particules, l'extrémité hydrophile du tensioactif étant adsorbée sur la surface des particules hydrophiles Le caractère hydrophobe favorise l'adsorption de la particule colloïdale solide sur la surface de la phase lipidique dispersée.

La quantité de tensioactif résiduelle dans la phase continue est toutefois de préférence inférieure à la concentration micellaire critique (CMC) c'est à dire à la concentration au-dessus de laquelle les molécules de tensioactif s'auto-associent sous forme de micelles ou agrégats sphériques. De préférence, la quantité de tensioactif est inférieure à celle nécessaire pour constituer une monocouche à la surface des particules.

En effet, le tensioactif ajouté ne s'adsorbe pas totalement à la surface des particules, mais est en équilibre avec la phase aqueuse de l'émulsion.

Or, lorsque la concentration résiduelle du tensioactif dans la phase aqueuse dépasse la concentration micellaire critique (CMC), le taux d'adsorption est maximal et la surface des particules est recouverte par une bicouche. Dans ces conditions, la surface des particules est totalement hydrophile et de ce fait, les particules minérales ne peuvent s'adsorber à la surface des gouttes d'huile.

Il est donc préférable que la concentration en tensioactif résiduelle dans la phase continue soit inférieure à la concentration micellaire critique de façon à ce que la surface des particules reste partiellement hydrophobe. L'homme de l'art peut aisément déterminer la quantité de tensioactif restée libre dans la phase continue. Une fois l'émulsion fabriquée, les phases continue et dispersée sont séparées par centrifugation et la concentration résiduelle de tensioactif dans la phase aqueuse est mesurée par extrait sec ou toute autre technique analytique.

Tout tensioactif, ionique ou non ionique, approprié pour la stabilisation d'émulsions peut être utilisé dans les compositions. Les tensioactifs cationiques et anioniques sont toutefois préférés.

Parmi ces tensioactifs, on préfère en particulier les alkylsulfates de sodium et les bromures d'alkyltriméthylammonium.

Il s'avère particulièrement avantageux de choisir un tensioactif de charge opposée à celle de la surface des particules. Ainsi, dans le cas des particules dont la surface est cationique, il est préférable d'utiliser un tensioactif anionique et vice-versa. Ce choix permet de favoriser l'adsorption du tensioactif sur la surface des particules par attraction électrostatique.

A titre d'exemple, il est préféré d'utiliser un tensioactif anionique comme le dodécylsulfate de sodium (SDS) en association avec des particules colloïdales dont la charge de surface positive est assurée par des groupes de type amine.

L'huile cristallisable forme une partie majoritaire de la phase lipidique dispersée et peut même être l'unique constituant de celle-ci. Généralement, elle représente 75 à 95% en poids, de préférence 80 à 90% en poids de la phase lipidique.

Le choix de l'huile cristallisable dépendra principalement de l'application envisagée de la composition. On peut notamment citer à titre d'huiles cristallisables les paraffines, triglycérides, collophanes, cires (alcools longs), huiles végétales hydrogénées ainsi que leurs mélanges et les bitumes synthétiques.

Des résultats très satisfaisants ont été obtenus avec une paraffine de chez Merck ayant un point de fusion entre 42 et 44°C [RN 8002-74-2].

Bien entendu, la phase lipidique solide peut comprendre également d'autres additifs tels que des principes actifs, colorants, pigments, agents odorants et texturants.

Cependant, la phase lipidique de la composition résultante est tout particulièrement au moins partiellement solide à température ambiante (20°C).

La phase continue de la composition comprend principalement de l'eau et éventuellement un alcool, tel que le méthanol, l'éthanol, l'isopropanol, ou le butanol, de préférence l'éthanol.

La phase aqueuse continue peut en outre également contenir d'autres agents habituellement présents, tels que des conservateurs ou des agents épaississants. Parmi les agent épaississants, on peut citer notamment les agents épaississants polysaccharidiques tels que la mousse d'Irlande, la gomme adragante, l'amidon et ses dérivés, la cellulose et ses dérivés, notamment l'hydroxyéthylpropylcellulose, l'hydroxybutylpropylcellulose, l'hydroxypropyl-éthylcellulose, l'hydroxyéthylcellulose ou la carboxyméthyl-cellulose, la gomme xanthane, la gomme de guar, les carraghénanes et les alginates.

L'invention concerne, selon un second aspect, un procédé de préparation de la composition selon l'invention.

Plus précisément, il est proposé un tel procédé comprenant les étapes consistant à :

a) mettre en contact une phase aqueuse et une phase lipidique comprenant majoritairement une huile cristallisable, à l'état liquide, en présence de particules solides colloïdales fonctionnalisées ; et

b) soumettre le mélange liquide ainsi obtenu à une agitation mécanique.

Les particules solides colloïdales peuvent être fonctionnalisées par des composés liés à la surface par des liaisons covalentes. Cela peut être réalisé par traitement préalable, en particulier par greffage chimique.

Il peut s'avérer parfois suffisant de fonctionnaliser les particules in situ, par exemple par simple mise en contact avec des composés susceptibles de s'adsorber à la surface, notamment des tensioactifs.

Les particules colloïdales solides sont de préférence ajoutées dans la phase aqueuse avant l'étape a).

Dans l'étape a), la phase lipidique en fusion peut être incorporée progressivement à la phase aqueuse chauffée contenant les particules.

De préférence, la phase lipidique est ajoutée à l'état liquide à la phase aqueuse amenée à la même température. Cependant, il est également possible de chauffer le mélange des deux phases ensemble jusqu'à fusion de la phase lipidique.

De préférence, l'étape b) est réalisée dans un dispositif émulsificateur de type Ultra-Turrax® ou Rayneri®.

L'Ultra-Turrax® est un homogénéiseur muni d'un système rotor/stator, à haut cisaillement qui permet d'obtenir des dispersions homogènes. Le rotor et le stator ont des formes dentelées et donnent naissance à un écoulement turbulent. Accessoirement, l'Ultra-Turrax® peut être remplacé par un agitateur de type hélice.

Il convient toutefois que l'agitation ne provoque pas le phénomène de cavitation afin d'éviter l'incorporation de bulles d'air dans l'émulsion finale. En effet, les particules solides peuvent aussi stabiliser la dispersion de bulles d'air dans l'eau.

Quel que soit le type d'agitation adopté, la distribution de taille de globules de la phase lipidique dans les émulsions obtenues est généralement étroite (U<40%). La distribution étroite de la taille moyenne de la phase dispersée est obtenue même en employant une technique d'émulsification en régime turbulent, tel qu'un Ultraturrax®, alors qu'en règle générale, la distribution d'une émulsion fabriquée en régime turbulent est très large.

Il est supposé que ce résultat résulte du couplage entre les phénomènes de fragmentation et de coalescence. En effet, l'énergie apportée en régime turbulent est suffisante pour que la collision entre deux gouttes fraîchement constituées puisse induire la coalescence. Au moment précis où cesse l'agitation, il est donc fort probable que la distribution des tailles de la phase dispersée soit large. Il est supposé qu'alors la quantité de silice initialement introduite est insuffisante pour recouvrir et ainsi stabiliser toute la surface engendrée pendant l'agitation. Il s'ensuit donc une coalescence des gouttes qui a pour conséquence de réduire la surface totale.

Contrairement au tensioactif, l'adsorption des particules solides colloïdales est irréversible (voir B.P. Binks, « Particles as surfactants - similarities and differences » Current Opinion in Colloid and Interface Science 7 (2002) p.21-41). En effet, elle fait intervenir un nombre considérable de chaînes hydrophobes par particule. Ainsi, si la surface diminue et si les particules ne se désorbent pas, le degré de couverture des surfaces augmente. On s'attend donc à ce que le processus de coalescence « s'auto-ralentisse » au cours du temps et finisse par être totalement bloqué. Dans ces conditions, la taille finale de la phase dispersée est fixée par la quantité de particules initialement introduites. Le processus ainsi décrit conduit alors à un resserrement de la distribution de taille de globules de la phase dispersée.

Selon un dernier aspect, l'invention concerne l'utilisation des compositions selon l'invention pour la préparation de produits cosmétiques, pharmaceutiques ou alimentaires, peintures, adhésifs et revêtements.

L'invention sera expliquée plus en détail au moyen des exemples ci-après, donnés à titre non limitatif, et des figures, lesquelles montrent :

Fig. 1 : un graphe de l'inverse du diamètre moyen D des globules de phase dispersée en fonction de la quantité de particules de la composition de l'exemple 1.

Fig. 2(a) : une microphotographie de la composition de l'exemple 1 avec 0,0182 g de particules de silice ;

Fig.2 (b) : la distribution de taille de globules de la phase dispersée de cette composition.

Fig. 3(a) : une microphotographie de la composition de l'exemple 3 ;

Fig. 3(b) : un graphe de l'inverse du diamètre moyen D des globules de phase dispersée en fonction de la quantité de particules de cette composition.

Fig. 4 : une vue de la composition de l'exemple 4 au microscope électronique à balayage.

Fig. 5 : une microphotographie de la composition de l'exemple 5.

## EXEMPLE 1

Dans un récipient approprié, on a introduit 2g de paraffine en bloc (RN 8002-74-2 de Merck, plage de fusion de 42 à 44°C). On ajoute la quantité nécessaire d'une solution aqueuse contenant des particules de silice d'un diamètre moyen de 21 nm (Aerosil 130, de chez Degussa), du tensioactif cationique (Bromure de cétyltriméthylammonium ou CTAB) à $5.10^{-5}$ M pour atteindre un poids total de 10 g de composition. Dans le cas présent, la surface des particules est anionique, ce qui justifie le choix du tensioactif cationique pour fonctionnaliser les surfaces. Le rapport massique tensioactif/silice est fixé à 0,0066. Le mélange a été ensuite chauffé jusqu'à fusion totale de la paraffine. Sous agitation manuelle lente, le mélange forme une émulsion. L'émulsion ainsi obtenue est fragmentée pendant 30s à 8000 tours par minute dans un dispositif Ultra-Turrax® muni d'une tête de type T25 (notation actuelle 25F) avec un axe en inox de type KV04/99 et chauffé à 60°C.

Les émulsions obtenues sont stables et leur distribution de taille n'est pas modifiée par un cycle fonte-recristallisation de la phase lipidique.


Evaluation de la taille de globules de la phase dispersée

La taille de globules de la phase dispersée ainsi que la largeur de la distribution ont été évaluées au moyen d'un granulomètre (Mastersizer S, Malvern) doté d'un système de mesure par diffusion statique de la lumière. L'échantillon (émulsion diluée) est soumis à un rayonnement lumineux. Les gouttes se comportent alors comme de petits dipôles oscillants qui rayonnent dans tout l'espace un champ électromagnétique diffusé : c'est le phénomène de diffusion de la lumière. La distribution angulaire de la lumière diffusée permet d'obtenir la distribution de tailles par l'intermédiaire de la théorie de Mie.

La mesure se fait à température ambiante en suivant les instructions d'utilisation du granulomètre. L'échantillon est prélevé et dilué dans une solution aqueuse contenant un tensioactif en concentration micellaire critique. L'utilisation du tensioactif permet d'éviter que les gouttes ne viennent se coller sur l'optique et altèrent la qualité du signal. La cellule de mesure est équipée d'une agitation et d'une circulation de l'échantillon pour éviter tout phénomène de crémage ou de sédimentation.

La grandeur principalement utilisée pour caractériser la granulométrie des émulsions est le diamètre moyen en volume D et l'indicateur de polydispersité de l'émulsion U.

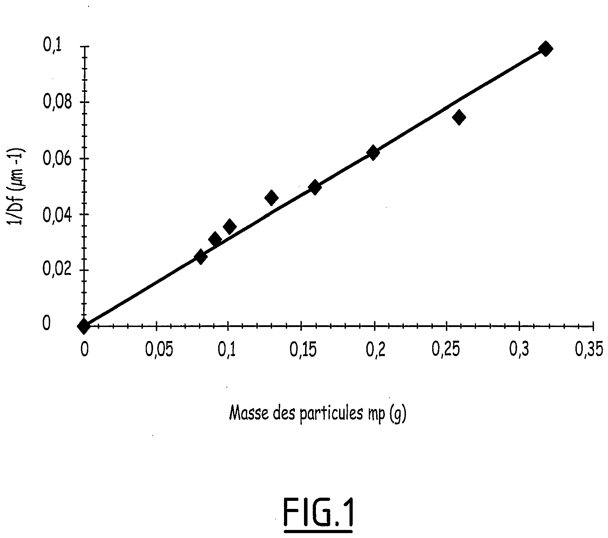

La figure 1 montre la relation de l'inverse du diamètre moyen D de la composition en fonction de la masse des particules de silice, le rapport massique tensioactif/silice étant de 0,0066. On constate que le diamètre diminue quand on augmente la masse de particules de silice.

La pente de la droite reportée sur la figure 1 permet d'extraire la valeur de la surface stabilisée par gramme de particules colloïdales de silice $S_f^{exp}$ comme étant de l'ordre de 25 $m^2g^{-1}$.

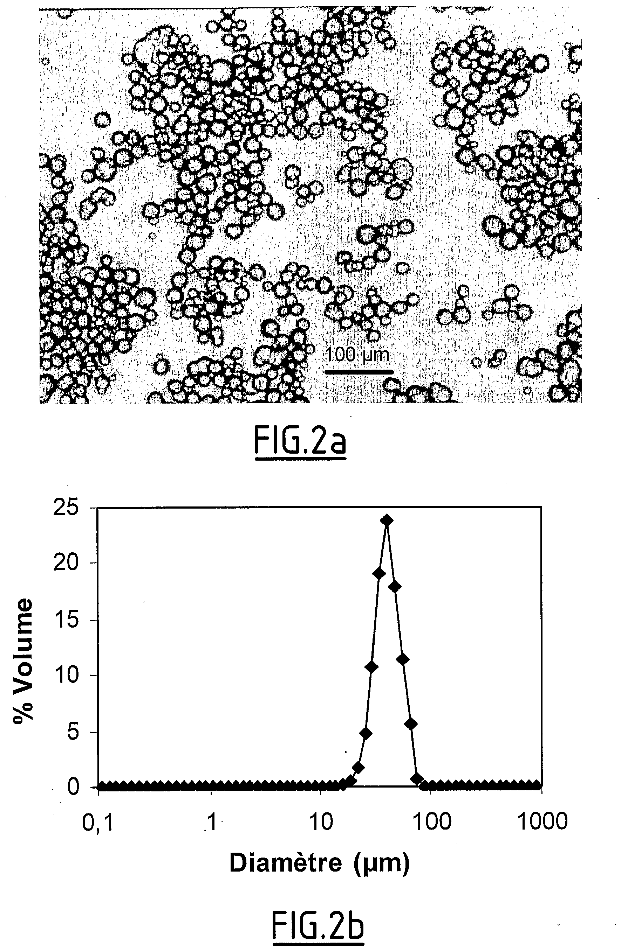

La figure 2 (a) montre l'aspect d'une émulsion contenant 0,0182g de particules de silice. La figure 2(b) montre la distribution de taille correspondante. La taille moyenne en volume de la phase dispersée est de 38 $\mu$m et l'indice de polydispersité est de 20,4%.

Ces compositions sont remarquablement stables au stockage sur une période de plusieurs mois.

La même émulsion, préparée sans particules colloïdales mais avec du tensioactif seul, n'est pas stable. Son aspect est rapidement grumeleux et si l'on chauffe l'émulsion, les gouttes de phase lipidique coalescent.

## EXEMPLE 2

Les essais suivants ont été réalisés afin d'évaluer la stabilité de la composition en fonction de la fraction en phase lipidique. La quantité de particules colloïdales solides et de tensioactif a été choisie afin de conserver un rapport constant entre tensioactif et particules solides d'une part ainsi qu'entre particules solides et phase lipidique d'autre part.

On a préparé une composition d'un poids total de 10 g comme à l'exemple 1, mais en variant les proportions des ingrédients comme indiqué dans le tableau ci-après :

**TABLEAU 1**

| Exemple | Paraffine [g] | Particules de silice [g] | Tensioactif [mol/l] | Rapport $m_{TA}/m_{silice}$ | Rapport $m_{silice}/m_{para}$ |
|---------|---------------|--------------------------|---------------------|-----------------------------|-------------------------------|
| 2A | 0,1 | 0,0009 | 0,0089 | 0,0066 | 0,0090 |
| 2B | 0,5 | 0,0045 | 0,0033 | 0,0066 | 0,0090 |
| 2C | 1 | 0,0090 | 0,0071 | 0,0066 | 0,0090 |
| 2D | 2 | 0,0182 | 0,159 | 0,0066 | 0,0091 |
| 2E | 3 | 0,0273 | 0,274 | 0,0066 | 0,0091 |
| 2F | 3,5 | 0,0319 | 0,345 | 0,0066 | 0,0091 |
| 2G | 4 | 0,0364 | 0,427 | 0,0066 | 0,0091 |

Les compositions sont toutes stables et résistent même à la centrifugation à 5000 g (g étant l'accélération de la pesanteur soit 9,8 ms$^{-2}$) pendant 5 minutes. Il est ainsi mis en évidence que les compositions stabilisées selon l'invention sont stables même pour une fraction importante de phase lipidique solide dispersée.

Par ailleurs, la taille moyenne de la phase dispersée de ces compositions est très proche, centrée autour de 33 micromètres. Ainsi, le rapport pondéral entre les particules de silice et la phase dispersée détermine la taille de globules de la phase dispersée de la composition stable obtenue.

## EXEMPLE 3

On a préparé une composition comme à l'exemple 1, à partir de 2,5 g de paraffine en bloc (RN 8002-74-2 de Merck), 0,086g de particules de silice (diamètre moyen 100 nm, greffées par de l'aminopropyltriéthoxysilane à raison de 15 chaînes par nm$^2$ de silice) et 0,0115 g de SDS (tensioactif anionique, les particules étant de natures cationiques), cette quantité correspondant à 2 molécules de SDS pour 3 chaînes à terminaison amine. On ajoute la quantité nécessaire d'un mélange eau/éthanol à 40% en masse d'eau pour atteindre 15g de poids total de la composition.

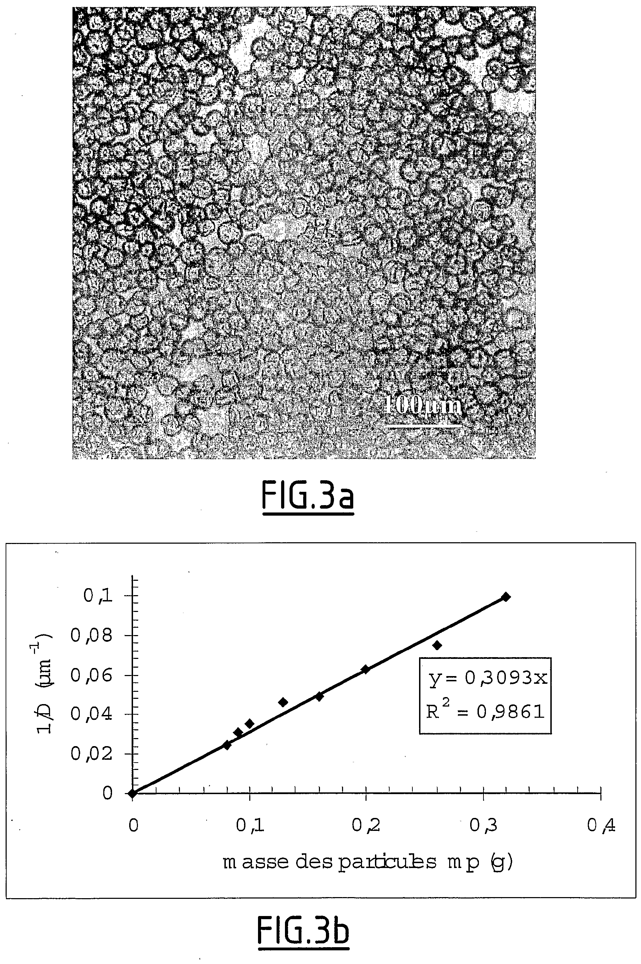

Cet essai est répété en variant la quantité de particules tout en conservant un rapport massique de tensioactif à particules de 0,134. En portant l'inverse du diamètre de ces compositions en fonction de la masse de particules, on obtient la courbe montrée sur la figure 3b. De la pente de la droite on extrait la valeur de $S_f^{exp}$ = 5,2 m$^2$/g.

Cette valeur est proche de la valeur théorique obtenue en supposant que les particules de rayon R constituent une monocouche dense à la surface des gouttes de la phase lipidique.

En effet, 1 g de silice peut stabiliser une surface $S_f^{th}$ égale à :

$$\frac{\pi R^2}{\frac{4}{3}\pi R^3 \rho C} = \frac{3}{4\rho R C}$$

où p est la masse volumique de la silice (2,2 10$^6$ g/m$^3$) et C est la compacité de l'arrangement des particules de silice à l'interface eau/paraffine ($C = \pi/2\sqrt{3} \approx 0,9$ pour un empilement hexagonal compact à 2 dimensions). Pour ce système, $S_f^{th}$ = 7,6 m$^2$/g avec un arrangement hexagonal compact des particules adsorbées à la surface de la paraffine. Cette valeur est du même ordre de grandeur que la valeur expérimentale.

On suppose que la phase dispersée subit des phénomènes de coalescence jusqu'à ce que les particules de silice recouvrent entièrement la surface de la phase dispersée. Un tel revêtement dense de la surface de la phase dispersée permet alors d'empêcher toute interaction et instabilité de type « edge-piercing ». Ainsi, les compositions selon l'invention permettent en outre de contrôler la taille de globules de la phase dispersée de la composition au moyen de la quantité de particules colloïdales solides ajoutées.

## EXEMPLE 4

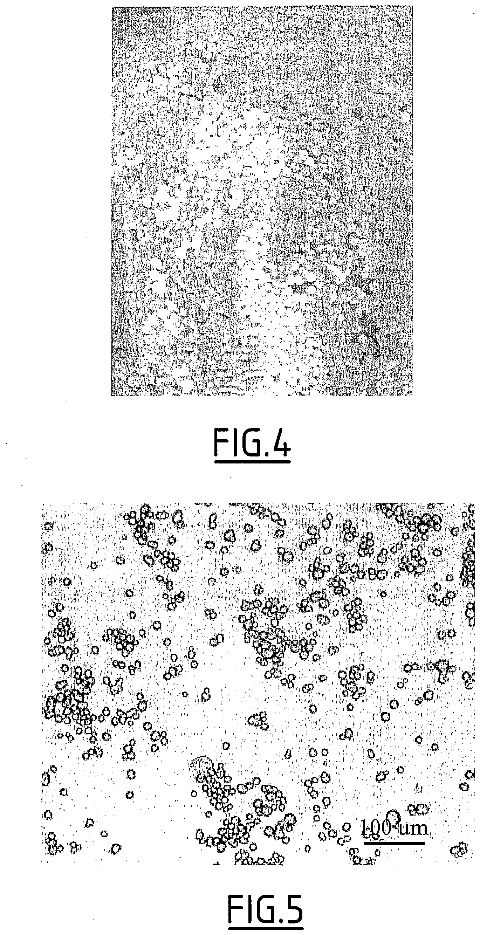

On a préparé une composition comme à l'exemple 1, à partir de 1 g de paraffine, 0,1 g de particules de silice non greffées de diamètre moyen de 1,1 $\mu$m et 610 $\mu$l d'une solution de CTAB à la concentration CMC/5 (CMC = 9.10$^{-4}$ M), le rapport massique de tensioactif à la silice étant égal à 410$^{-4}$. La photographie en microscopie électronique à balayage (MEB) (figure 4) montre bien la présence des particules à l'interface et leur arrangement compact hexagonal.

## EXEMPLE 5

Pour une masse totale de 10 g, 2 g de paraffine sont dispersés à chaud dans un mélange de 7,7 g d'eau et 0,3 g de particules de silice de 340 nm de diamètre préalablement greffées par du n-octyltriéthoxysilane à raison de 5 chaînes par nm$^2$ de silice.

Cette émulsion est stable et résiste bien à plusieurs cycles de fonte-cristallisation de la paraffine. Une photographie de cette émulsion est indiquée à la figure 5. La taille moyenne de la phase dispersée est d'environ 20 $\mu$m.

Ainsi, les compositions décrites, comprenant une phase aqueuse continue et une phase lipidique dispersée à base d'huile cristallisable sont stables, même concentrées, grâce à la présence de particules colloïdales solides.

La phase dispersée présente en outre l'avantage d'être monodisperse, même lorsque la composition a été préparée au moyen d'un écoulement turbulent.

De plus, la taille de globules de la phase dispersée est contrôlée très simplement par la quantité introduite de particules colloïdales solides.

Ce type de composition est en outre simple à fabriquer et peut être utile pour la préparation de compositions cosmétiques, pharmaceutiques, alimentaires ou peintures, adhésifs et revêtements.

**Revendications**

1. Composition comprenant une phase aqueuse continue, une phase lipidique dispersée au moins partiellement solide et des particules solides colloïdales à titre d'agent stabilisant, ladite phase dispersée présentant un indice de polydispersité inférieur à 40%, ladite phase lipidique dispersée comprenant de 50 à 100 % en poids d'huile cristallisable par rapport au poids total en phase lipidique, les particules solides colloïdales étant fonctionnalisées en surface par un composé comportant un groupement hydrophobe ou par un agent tensioactif dont la concentration finale dans la phase continue de la composition est inférieure à la concentration micellaire critique.

2. Composition selon la revendication 1, dans laquelle la fraction volumique de la phase lipidique est comprise entre 0,01% et 50%.

3. Composition selon l'une des revendications 1 à 2, dans laquelle la phase lipidique dispersée se présente sous la forme d'objets dont la taille moyenne en volume est comprise entre 1 et 500 $\mu$m.

4. Composition selon l'une des revendications 1 à 3, comprenant 0,01 à 10% en poids de particules solides colloïdales.

5. Composition selon l'une des revendications 1 à 4, dans laquelle les particules solides colloïdales sont minérales ou organique, en particulier polymériques.

6. Composition selon l'une des revendications 1 à 5, dans laquelle les particules solides colloïdales ont une taille moyenne comprise entre 10 et 1000 nm.

7. Composition selon la revendication 6, dans laquelle le groupement hydrophobe comprend une chaîne alkyle comportant au moins 3, de préférence 3 à 20 atomes de carbone.

8. Composition selon la revendication 6 ou 7, dans laquelle le composé comportant un groupement hydrophobe est un trialkoxysilane de formule R-Si-(OR')$_3$, dans laquelle R est un alkyle linéaire ou ramifié en C$_1$ à C$_{12}$ portant éventuellement un groupe amine, et R', identique ou différent, est un groupe alkyle linéaire ou ramifié en C$_1$ à C$_{12}$.

9. Composition selon l'une des revendications 1 à 8, dans laquelle les particules solides colloïdales sont des particules d'oxyde de silicium ou d'oxyde de titane.

10. Composition selon la revendication 1, dans laquelle l'agent tensioactif est un tensioactif ionique ou non-ionique.

11. Composition selon l'une des revendications 1 à 10, dans laquelle l'huile cristallisable est choisie parmi les paraffines, triglycérides, collophanes, cires (alcools longs), huiles végétales hydrogénées et leurs mélanges, bitumes synthétiques.

12. Composition selon l'une des revendications 1 à 11, dans laquelle la phase aqueuse continue comprend un alcool.

13. Composition selon l'une des revendications 1 à 12, dans laquelle la phase aqueuse comprend un agent épaississant.

14. Procédé de préparation d'une composition selon l'une des revendications 1 à 13 comprenant les étapes consistant à :

   a) mettre en contact une phase aqueuse et une phase lipidique comprenant majoritairement une huile cristallisable, à l'état liquide, en présence de particules solides colloïdales fonctionnalisées ; et
   b) soumettre l'émulsion ainsi obtenue à une agitation mécanique.

15. Procédé selon la revendication 14, dans lequel les particules solides colloïdales sont fonctionnalisées in situ par adsorption.

16. Procédé selon la revendication 15, dans lequel les particules solides colloïdales sont fonctionnalisées par greffage chimique.

**17.** Procédé selon l'une des revendications 14 à 16, dans lequel l'étape b) est réalisée dans un homogénéiseur muni d'un système rotor/stator de formes dentelées et donnant naissance à un écoulement turbulent ou un agitateur du type hélice.

**18.** Utilisation de la composition selon la revendication 1 à 13 pour la préparation de produits cosmétiques, pharmaceutiques ou alimentaires, peintures, adhésifs et revêtements.

**Patentansprüche**

**1.** Zusammensetzung, enthaltend eine kontinuierliche wässrige Phase, eine dispergierte Lipidphase, mindestens teilweise fest, und kolloidale Feststoffpartikel als Stabilisierungsmittel, wobei die dispergierte Phase einen Polydispersitätsindex kleiner als 40% aufweist, die dispergierte Lipidphase von 50 bis 100 Gew.-% eines kristalliserbaren Öls, bezogen auf das Gesamtgewicht der Lipidphase, enthält, die kolloidalen Feststoffpartikel an der Oberfläche durch eine eine hydrophobe Gruppe aufweisende Verbindung oder durch ein oberflächenaktives Mittel, dessen Endkonzentration in der kontinuierlichen Phase der Zusammensetzung kleiner als die kritische Mizellkonzentration ist, funktionalisiert sind.

**2.** Zusammensetzung nach Anspruch 1, bei der die Volumenfraktion der Lipidphase zwischen 0,01% und 50% liegt.

**3.** Zusammensetzung nach einem der Ansprüche 1 bis 2, bei der die dispergierte Lipidphase in Form von Objekten ausgebildet ist, deren mittlere Volumengröße zwischen 1 und 500 $\mu$m liegt.

**4.** Zusammensetzung nach einem der Ansprüche 1 bis 3, enthaltend 0,01 bis 10 Gew.-% an kolloidalen Feststoffpartikeln.

**5.** Zusammensetzung nach einem der Ansprüche 1 bis 4, bei der die kolloidalen Feststoffpartikel mineralisch oder organisch, insbesondere Polymere sind.

**6.** Zusammensetzung nach einem der Ansprüche 1 bis 5, bei der die kolloidalen Feststoffpartikel eine mittlere Größe zwischen 10 und 1000 nm aufweisen.

**7.** Zusammensetzung nach Anspruch 6, bei der die hydrophobe Gruppe eine Alkylkette enthält, die mindestens 3, vorzugsweise 3 bis 20 Kohlenstoffatome aufweist.

**8.** Zusammensetzung nach Anspruch 6 oder 7, bei der die eine hydrophobe Gruppe aufweisende Verbindung ein Trialkoxysilan der Formel R-Si-(OR')$_3$ ist, bei der R ein lineares oder verzweigtes $C_1$ bis $C_{12}$ Alkyl ist, das gegebenenfalls eine Amingruppe trägt, und R', identisch oder unterschiedlich, eine lineare oder verzweigte $C_1$ bis $C_{12}$ Alkylgruppe ist.

**9.** Zusammensetzung nach einem der Ansprüche 1 bis 8, bei der die kolloidalen Feststoffpartikel Siliciumoxidpartikel oder Titanoxidpartikel sind.

**10.** Zusammensetzung nach Anspruch 1, bei der das oberflächenaktive Mittel ein ionisches oder nichtionisches Tensid ist.

**11.** Zusammensetzung nach einem der Ansprüche 1 bis 10, bei der das kristallisierbare Öl ausgewählt ist aus Paraffinen, Triglyzeriden, Kollophanen, Wachsen (höheren Alkoholen), hydrierten Pflanzenölen und ihren Mischungen, synthetische Bitumen.

**12.** Zusammensetzung nach einem der Ansprüche 1 bis 11, bei der die kontinuierliche wässrige Phase ein Alkohol enthält.

**13.** Zusammensetzung nach einem der Ansprüche 1 bis 12, bei der die wässrige Phase ein Verdickungsmittel enthält.

**14.** Verfahren zur Herstellung einer Zusammensetzung nach einem der Ansprüche 1 bis 13, die Schritte umfassend, die bestehen aus:

a) Inkontaktbringen einer wässrigen Phase und einer Lipidphase, die hauptsächlich ein kristallisierbares Öl im flüssigen Zustand bei Vorhandensein von funktionalisierten kolloidalen Feststoffpartikeln enthält, und
b) Unterwerfen der so erhaltenen Emulsion einem mechanischen Umrühren.

**15.** Verfahren nach Anspruch 14, bei dem die kolloidalen Feststoffpartikel in situ durch Adsorption funktionalisiert werden.

**16.** Verfahren nach Anspruch 15, bei dem die kolloidalen Feststoffpartikel durch chemisches Pfropfen funktionalisiert werden.

**17.** Verfahren nach einem der Ansprüche 14 bis 16, bei dem der Schritt b) in einem Homogenisierer, der mit einem eine turbulente Strömung erzeugenden System Rotor/Stator von gezahnter Form ausgerüstet ist, oder einem Propellerrührer hergestellt wird.

**18.** Verwendung einer Zusammensetzung nach Anspruch 1 bis 13 für die Herstellung von kosmetischen pharmazeutischen Lebensmittelprodukten, Farben, Klebstoffen und Beschichtungen.

## Claims

**1.** Composition comprising a continuous aqueous phase, an at least partially solid dispersed lipid phase and solid colloidal particles as stabilizing agent, said dispersed phase having a polydispersity index of less than 40%, said dispersed lipid phase comprising from 50% to 100% by weight of crystallizable oil relative to the total weight of the lipid phase, the solid colloidal particles being surface-functionalized with a compound comprising a hydrophobic group or with a surfactant, the final concentration of which in the continuous phase of the composition is less than the critical micelle concentration.

**2.** Composition according to Claim 1, in which the volume fraction of the lipid phase is between 0.01% and 50%.

**3.** Composition according to either of Claims 1 and 2, in which the dispersed lipid phase is in the form of objects of which the volume-average size is between 1 and 500 $\mu$m.

**4.** Composition according to one of Claims 1 to 3, comprising 0.01% to 10% by weight of solid colloidal particles.

**5.** Composition according to one of Claims 1 to 4, in which the solid colloidal particles are mineral or organic, in particular polymeric.

**6.** Composition according to one of Claims 1 to 5, in which the solid colloidal particles have an average size of between 10 and 1000 nm.

**7.** Composition according to Claim 6, in which the hydrophobic group comprises an alkyl chain comprising at least 3, preferably 3 to 20, carbon atoms.

**8.** Composition according to Claim 6 or 7, in which the compound comprising a hydrophobic group is a trialkoxysilane of formula R-Si-(OR')$_3$, in which R is a linear or branched $C_1$ to $C_{12}$ alkyl optionally bearing an amine group, and R', which may be identical or different, is a linear or branched $C_1$ to $C_{12}$ alkyl group.

**9.** Composition according to one of Claims 1 to 8, in which the solid colloidal particles are silicon oxide particles or titanium oxide particles.

**10.** Composition according to Claim 1, in which the surfactant is an ionic or non-ionic surfactant.

**11.** Composition according to one of Claims 1 to 10, in which the crystallizable oil is chosen from paraffins, triglycerides, rosins, waxes (long alcohols), hydrogenated vegetable oils and mixtures thereof, synthetic bitumens.

**12.** Composition according to one of Claims 1 to 11, in which the continuous aqueous phase comprises an alcohol.

**13.** Composition according to one of Claims 1 to 12, in which the aqueous phase comprises a thickener.

**14.** Method for preparing a composition according to one of Claims 1 to 13, comprising the steps consisting in:

a) bringing into contact an aqueous phase and a lipid phase comprising predominantly a crystallizable oil, in the liquid state, in the presence of functionalized solid colloidal particles; and
b) subjecting the emulsion thus obtained to mechanical stirring.

**15.** Method according to Claim 14, in which the solid colloidal particles are functionalized in situ by adsorption.

**16.** Process according to Claim 15, in which the solid colloidal particles are functionalized by chemical grafting.

**17.** Method according to Claims 14 to 16, in which step b) is carried out in a homogenizer equipped with a rotor/stator system which has serrated shapes and gives rise to a turbulent flow, or a propeller-type stirrer.

**18.** Use of the composition according to Claim 1 to 13, for preparing cosmetic, pharmaceutical or food products, paints, adhesives and coatings.

FIG.1

## FIG.2a

## FIG.2b

## FIG.3a

$$y = 0,3093x$$
$$R^2 = 0,9861$$

m asse des particules m p (g)

## FIG.3b

FIG.4

FIG.5

## RÉFÉRENCES CITÉES DANS LA DESCRIPTION

**Documents brevets cités dans la description**

- EP 0987002 A

**Littérature non-brevet citée dans la description**

- **S.U. PICKERING.** *J. Chem. Soc.,* 1907, vol. 91, 2001-2021
- **STÖBER, W. ; FINK, A. ; BOHN, E.** *J. Coll. Interface Sci.,* 1968, vol. 26, 62-69
- **B.P. BINKS.** Particles as surfactants - similarities and differences. *Current Opinion in Colloid and Interface Science,* 2002, vol. 7, 21-41